# EUROPEAN PATENT APPLICATION

(11) **EP 4 596 645 A1**
(43) Date of publication of application: **06.08.2025**
(21) Application number: 23872267.2
(22) Date of filing: 25.09.2023
(51) Int. Cl.: C09D 179/08, C09D 7/61, C09D 7/65, C10M 125/02, C10M 133/42, C10M 147/00

(54) **COATING COMPOSITION FOR ENDOSCOPES, LUBRICATION MEMBER FOR ENDOSCOPES, METHOD FOR PRODUCING LUBRICATION MEMBER FOR ENDOSCOPES, FLEXIBLE TUBE FOR ENDOSCOPES, AND ENDOSCOPE**

(30) Priority: 29.09.2022 JP 2022156219
(71) Applicant: FUJIFILM Corporation, Tokyo 106-8620 (JP)
(72) Inventor: NAKAI, Yoshihiro, Ashigarakami-gun, Kanagawa 258-8577 (JP)
(74) Representative: HGF
(86) International application number: PCT/JP2023/034750
(87) International publication number: WO 2024/071044

(57) **Abstract**

Provided are a coating composition for an endoscope, the coating composition including a solid lubricant, a thermosetting resin, a solvent, and a nonionic dispersing agent, in which the nonionic dispersing agent is a compound constituted by a carbon element, a hydrogen element, and at least one of an oxygen element or a nitrogen element; a lubricating member that is suitable as a member for an endoscope and that has been subjected to a coating treatment with the coating composition and a method for manufacturing the lubricating member; and a flexible tube for an endoscope and an endoscope that use the lubricating member.

## Description

### BACKGROUND OF THE INVENTION

### 1. Field of the Invention

The present invention relates to a coating composition for an endoscope, a lubricating member for an endoscope, a method for manufacturing a lubricating member for an endoscope, a flexible tube for an endoscope, and an endoscope.

### 2. Description of the Related Art

Endoscopes are medical devices that observe the inside of a patient's body (digestive tract, trachea, body cavity, and the like) and are configured to include a treatment tool and to be able to perform various treatments in a state of being inserted into the body.

Such an endoscope includes a bendable bending part on a tip side of an insertion part, so that an observation visual field direction can be freely changed. For example, in the bending part, a wire formed of a twisted wire bundle of metal wires made of stainless steel or the like is inserted into the endoscope insertion part. The wire is pulled by controlling an operating unit at a hand, so that the bending part is bent in a desired direction. This wire is inserted into a wire guide provided on an inner wall of the endoscope insertion part and is fixed to be slidable along the inner wall of the endoscope insertion part. The operation itself of the bending part using the wire is a function included in a general endoscope, and is disclosed in, for example, WO2018/034021A. As a general configuration of the wire and the wire guide in the bending part, for example, Fig. 2 of WO2018/034021A can be referred to.

As a result of a bending operation of the bending part, friction occurs between the above-described wire and wire guide, which causes a problem of abrasion of the wire. In order to reduce the abrasion, a coating treatment using a solid lubricant composed of inorganic particles or organic particles (for example, JP2004-208962A) is usually performed on the wire surface.

The coating treatment with the solid lubricant is generally performed by preparing a coating composition (coating agent) in which a solid lubricant is dispersed in a medium, and applying the coating composition to the surface of a to-be-coated member by spraying or the like. For the coating composition, a solvent is used as a medium, and a resin is usually blended as a binder. For such a coating composition in which a solid lubricant is dispersed in a medium, a technology of blending a dispersing agent or a surfactant in order to improve the dispersion stability of solid lubricant particles has been studied.

For example, for compositions containing a solid lubricant, a thermosetting resin as a binder, and a solvent, a composition in which a nitrogen-containing compound is blended as a dispersing agent is described in JP2010-116336A, and a composition in which a fluorine-containing surfactant is blended as a dispersing agent is described in WO2020/250676A.

### SUMMARY OF THE INVENTION

In a case where the to-be-coated member on which a coating layer including a solid lubricant is formed is a member for an endoscope represented by the above-described wire, it is required that the to-be-coated member can smoothly slide for a long period of time (sliding durability). As a result of studies performed by the present inventor, it has been found that the sliding durability is greatly affected by the performance in which the coating state of the wire with the coating layer can be sufficiently maintained against the repetition of the bending operation of the wire (bending durability).

It has also been found that the member for an endoscope on which the coating layer including the solid lubricant is formed is often subjected to a sterilization treatment such as low-temperature gas sterilization (H₂O₂ gas sterilization) having a strong chemical reactivity, and there is a problem that the coating layer is deteriorated by the sterilization treatment.

An object of the present invention is to provide a coating composition for an endoscope, which has good dispersion stability of a solid lubricant and can be applied onto a surface of a member for an endoscope to form a coating layer, thereby imparting good bending durability and good sterilization resistance to the member for an endoscope. Another object of the present invention is to provide a lubricating member for an endoscope in which a coating treatment is performed using the above-described coating composition, and a method for manufacturing the lubricating member for an endoscope. Still another object of the present invention is to provide a flexible tube for an endoscope and an endoscope that use the above-described lubricating member for an endoscope.

That is, the above-described objects of the present invention are achieved by the following means.
[1] A coating composition for an endoscope includes a solid lubricant, a thermosetting resin, a solvent, and a nonionic dispersing agent, wherein the nonionic dispersing agent is a compound constituted by a carbon element, a hydrogen element, and at least one of an oxygen element or a nitrogen element.
[2] In the coating composition for an endoscope according to [1], the dispersing agent includes a compound having an alkyleneoxy group.
[3] In the coating composition for an endoscope according to [2], the compound having an alkyleneoxy group has an aromatic ring group.
[4] In the coating composition for an endoscope according to [2] or [3], the alkyleneoxy group includes a propyleneoxy group.
[5] In the coating composition for an endoscope according to any one of [1] to [4], the dispersing agent has a molecular weight of 100 to 2,000.
[6] In the coating composition for an endoscope according to any one of [1] to [5], the thermosetting resin includes a polyamideimide resin.
[7] In the coating composition for an endoscope according to [6], the polyamideimide resin includes a structural unit represented by Formula (1) below as a constituent component of polyamideimide, 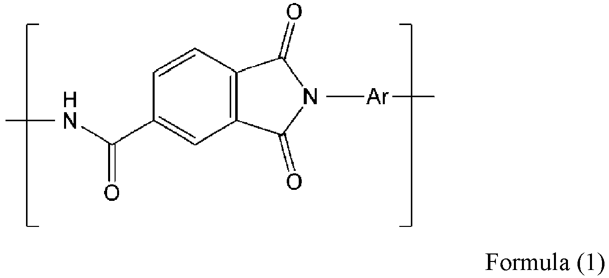 where Ar represents a divalent group having an aromatic ring.
[8] In the coating composition for an endoscope according to [7], Ar in the Formula (1) represents a phenylene group, a diphenylenemethane group, a diphenylenepropane group, a diphenylene ether group, or a diphenylene amine group.
[9] In the coating composition for an endoscope according to any one of [6] to [8], the polyamideimide resin has a weight-average molecular weight of 10,000 to 100,000.
[10] In the coating composition for an endoscope according to any one of [1] to [9], the solid lubricant includes inorganic particles, and the inorganic particles include at least one of graphite, fluorinated graphite, graphene, carbon nanotube, or boron nitride.
[11] In the coating composition for an endoscope according to [10], the inorganic particles have a particle diameter of 0.5 to 70 µm.
[12] In the coating composition for an endoscope according to [11], the inorganic particles have a particle diameter of 1.0 to 50 µm.
[13] In the coating composition for an endoscope according to any one of [1] to [12], the solid lubricant includes organic particles, and the organic particles include at least one of fluororesin, melamine cyanurate, or benzoguanamine.
[14] In the coating composition for an endoscope according to [13], the organic particles have a particle diameter of 0.5 to 30 µm.
[15] In the coating composition for an endoscope according to [14], the organic particles have a particle diameter of 1.0 to 10 µm.
[16] In the coating composition for an endoscope according to any one of [1] to [15], the solvent includes at least one of N-methyl-2-pyrrolidone, N,N-dimethylacetamide, N,N-dimethylformamide, hexamethylphosphoric triamide, γ-butyrolactone, or 1,3-dimethyl-2-imidazolidinone.
[17] A lubricating member for an endoscope is subjected to a coating treatment with the coating composition for an endoscope according to any one of [1] to [16].
[18] In the lubricating member for an endoscope according to [17], the lubricating member for an endoscope is a lubricating wire for an endoscope.
[19] A method for manufacturing a lubricating member for an endoscope includes applying the coating composition for an endoscope according to any one of [1] to [16] onto a surface of a member for an endoscope.
[20] A flexible tube for an endoscope includes the lubricating member for an endoscope according to [17] or [18].
[21] An endoscope includes the flexible tube for an endoscope according to [20].

In the present invention, a structure in which a substitution or unsubstitution is not specified means that the structure may have any substituent without impairing the desired effects.

In the present invention, the numerical range expressed using "to" means a range including numerical values before and after "to" as the lower and upper limit values.

The coating composition for an endoscope according to the present invention has good dispersion stability of a solid lubricant and can be applied onto a surface of a member for an endoscope to form a coating layer, thereby imparting good bending durability and high sterilization resistance to the member for an endoscope.

The lubricating member for an endoscope according to the present invention has good bending durability and high sterilization resistance. According to the method for manufacturing a lubricating member for an endoscope of the present invention, it is possible to obtain a lubricating member for an endoscope having good bending durability and high sterilization resistance. The flexible tube for an endoscope and the endoscope according to the present invention incorporate the lubricating member for an endoscope according to the present invention, and the lubricating member has good bending durability and high sterilization resistance. That is, the flexible tube for an endoscope and the endoscope according to the present invention have good bending durability and high sterilization resistance.

### BRIEF DESCRIPTION OF THE DRAWINGS

Fig. 1 is an external view illustrating a configuration of an electronic endoscope according to an embodiment; and
Fig. 2 is an explanatory diagram of a sliding property test in Examples.

### DESCRIPTION OF THE PREFERRED EMBODIMENTS

### Coating composition for endoscope

The coating composition for an endoscope of the present invention (hereinafter, also referred to as a composition of the present invention) includes a solid lubricant, a thermosetting resin, a solvent, and a nonionic dispersing agent (N) described later.

### Solid lubricant

To the solid lubricant included in the composition according to the present invention, those that are generally used as a solid lubricant can be widely applied. Therefore, the solid lubricant used in the present invention may be inorganic particles or organic particles, or may be used in a combination thereof. The "solid" in the solid lubricant means that the solid lubricant is in a solid state at 25°C.

Examples of the material for the solid lubricant composed of inorganic particles (also referred to as "inorganic solid lubricant" in the present invention) include graphite, fluorinated graphite, graphene, carbon nanotube, and boron nitride. One or two or more of them are preferably used.

In a case where the solid lubricant is composed of inorganic particles, the particle diameter thereof can be set to, for example, 0.5 to 70 µm.From the viewpoint of further improving the bending durability, the particle diameter is preferably 1.0 to 50 µm.From the viewpoint of further improving the liquid dispersion stability, the bending durability, and the sterilization resistance against hydrogen peroxide gas, the particle diameter is more preferably 3.5 to 40 µm, still more preferably 4.0 to 30 µm, and even more preferably 4.0 to 25 µm.

In the present invention or the specification, the particle diameter of the solid lubricant means an arithmetic average value of 100 maximum diameters (the maximum distance from a point on an outer periphery of the observed particle to another point) in a plan view of 100 particles randomly observed with an electron microscope.

Examples of the material for the solid lubricant composed of organic particles (also referred to as "organic solid lubricant" in the present invention) include fluororesin, melamine cyanurate, and benzoguanamine, and it is preferable to include at least one of them. Examples of the fluororesin include polytetrafluoroethylene (PTFE), polychlorotrifluoroethylene (PCTFE), polyvinylidene fluoride (PVDF), and polyvinyl fluoride (PVF). Among them, PTFE is suitable.

In a case where the solid lubricant is composed of organic particles, the particle diameter thereof can be set to 0.5 to 30 µm, can also be set to 1.0 to 10 µm, or is preferably 2.0 to 9 µm.

The composition according to the present invention preferably includes at least inorganic particles as the solid lubricant, and more preferably includes inorganic particles and organic particles.

In a case where the composition according to the present invention contains the inorganic particles and the organic particles as the solid lubricant, the mass ratio of the contents of both particles is preferably [inorganic particles]/[organic particles] ≥ 0.3, and more preferably [inorganic particles]/[organic particles] ≥ 0.5. The upper limit value thereof is preferably [inorganic particles]/[organic particles] ≤ 3.0, and more preferably [inorganic particles]/[organic particles] ≤ 2.0. That is, 0.3 ≤ [inorganic particles]/[organic particles] ≤ 3.0 is preferable, and 0.5 ≤ [inorganic particles]/[organic particles] ≤ 2.0 is more preferable.

In the composition according to the present invention, the content of the solid lubricant is preferably 10% to 50% by mass, more preferably 10% to 40% by mass, and still more preferably 10% to 30% by mass.

### Nonionic dispersing agent (N)

The nonionic dispersing agent included in the composition according to the present invention is a compound constituted by a carbon element, a hydrogen element, and at least one element of an oxygen element or a nitrogen element. This nonionic dispersing agent is also referred to as a "nonionic dispersing agent (N)" in the present specification.

In the present invention, the "nonionic dispersing agent" means a dispersing agent that does not substantially have either an acid value or an amine value, but that has a nonionic hydrophilic group. Therefore, the nonionic dispersing agent (N) has an acid value of 15 mgKOH/g or less and an amine value of 15 mgKOH/g or less.

The acid value is a value measured by an indicator titration method, a potentiometric titration method, or an indicator photometric titration method in conformity with Japanese Industrial Standards (JIS) K 2501 (2003). When the compound does not have a carboxy group or a salt thereof, the acid value is set to 0 mgKOH/g.

The amine value is a value measured by an indicator titration method or a potentiometric titration method in conformity with JIS K 2501 (2003). When the compound does not have any of amino groups (unsubstituted, primary, or secondary) or ammonio groups (unsubstituted, primary to tertiary), the amine value is set to 0 mgKOH/g.

In the present invention, the expression "the compound 'is constituted by a carbon element, a hydrogen element, and at least one element of an oxygen element or a nitrogen element'" means that the compound has a carbon element, a hydrogen element, and at least one element of an oxygen element or a nitrogen element, and does not include, as constituent elements, elements other than the carbon element, the hydrogen element, the oxygen element, and the nitrogen element.

For example, the nonionic dispersing agent (N) does not include a fluorine element. As shown in Comparative Example 2 described below, in a case where the nonionic dispersing agent including a fluorine element is used, the extremely high hydrophobicity (low surface energy) thereof reduces the adhesive force between a coating layer formed using the coating composition for an endoscope and a to-be-coated member (substrate), which impairs the bending durability.

On the other hand, this means that the nonionic dispersing agent (N) may include a small amount of an element other than the carbon element, the hydrogen element, the oxygen element, and the nitrogen element (excluding the fluorine element) without impairing the effects of the present invention. Examples of elements that may be included in the nonionic dispersing agent (N) include a sulfur element, a silicon element, a phosphorus element, a boron element, and a chlorine element.

The total of the proportion (at%) of carbon atoms, oxygen atoms, and nitrogen atoms relative to the total number of atoms other than hydrogen atoms constituting the nonionic dispersing agent (N) is preferably 97 at% or more, more preferably 99.0 at% or more, still more preferably 99.5 at% or more, and particularly preferably 100 at%.

The nonionic dispersing agent (N) is preferably a compound that does not include a nitrogen element as a constituent element, that is, a compound constituted by a carbon element, a hydrogen element, and an oxygen element.

The nonionic dispersing agent (N) acts on the solid lubricant to enhance the dispersibility of the solid lubricant in a solvent (medium). It is presumed that the nonionic dispersing agent (N) interacts with the medium in a compatible manner at a nonionic water-soluble group, and adsorbs to the solid lubricant at the remaining portion to function as a dispersing agent of the solid lubricant.

The connecting portion between the nonionic water-soluble group and the remaining portion responsible for the adsorption to the solid lubricant is not particularly limited as long as the effects of the present invention are not impaired. Examples of the connecting portion include an ether bond, an ester bond, an amide bond, and a urethane bond. Among them, an ether bond or an ester bond is preferable and an ether bond is more preferable.

Examples of the nonionic dispersing agent (N) that can be applied include a polyoxyalkylene-based dispersing agent, an amide-based dispersing agent, a polyester-based high-molecular-weight dispersing agent, an acrylic-based high-molecular-weight dispersing agent, a polyurethane-based high-molecular-weight dispersing agent, an alkyl glucoside, a sucrose fatty acid ester, a sorbitan fatty acid ester, polyvinyl alcohol (PVA), methyl cellulose, ethyl cellulose, and gelatin.

Examples of the nonionic water-soluble group include alkyleneoxy groups (preferably, a polyalkyleneoxy group) such as a polyoxyethylene group, a polyoxypropylene group, and a polyoxybutylene group, polyglycerin groups, and sorbitan groups. The nonionic dispersing agent (N) preferably has one or two or more of these groups.

**In** particular, the nonionic dispersing agent (N) preferably includes a compound having an alkyleneoxy group.

**In** the present invention, the "alkyleneoxy group" has a meaning of including not only a monoalkyleneoxy group having one alkyleneoxy structure but also a polyalkyleneoxy group having a polyalkyleneoxy structure in which two or more alkyleneoxy structures are repeated.

**In** the polyalkyleneoxy group, the average repeating number of the alkyleneoxy structure is preferably 2 to 50 and more preferably 2 to 20. **In** the present invention, the "average" in the average repeating number means a number average, and is a value measured and calculated by, for example, gel permeation chromatography (GPC) or nuclear magnetic resonance (NMR) spectrometry.

**In** the alkyleneoxy group, the number of carbon atoms in all the alkylene group constituting the alkyleneoxy structure is preferably an integer of 2 to 10, more preferably an integer of 2 to 6, and still more preferably 2 or 3. Examples of the alkyleneoxy structure in the alkyleneoxy group include an ethyleneoxy structure, a propyleneoxy structure, and a butyleneoxy structure. The polyalkyleneoxy group may have two or more alkyleneoxy structures.

From the viewpoint of affinity for a solvent, it is preferable that two adjacent oxygen atoms constituting the polyalkyleneoxy structure are connected by an ethylene group (-C-C-) or a 1,3-propanediyl group (-C-C-C-). **In** -C-C- or -C-C-C-, C may be further substituted with an alkyl group (carbon atom).

From the viewpoint of further improving the sterilization resistance against hydrogen peroxide gas, the alkyleneoxy structure in the alkyleneoxy group is preferably a propyleneoxy structure, that is, the alkylene group in the alkyleneoxy group is preferably a propylene group. Here, the propylene group constituting the propyleneoxy group may be any of a 1,3-propanediyl group and a 1,2-propanediyl group, and is preferably a 1,2-propanediyl group. The nonionic dispersing agent (N) having an alkyleneoxy group may further have an alkyleneoxy structure other than the propyleneoxy structure (for example, an ethyleneoxy structure).

From the viewpoint of affinity for a solvent, the alkyleneoxy group is preferably a group having a polyoxyalkylene structure, and more preferably a group having a polypropyleneoxy structure. **In** a case where the alkyleneoxy group is a group having a polypropyleneoxy structure, the alkyleneoxy group may further have a polyalkyleneoxy structure other than the polypropyleneoxy structure (for example, a polyethyleneoxy structure).

The above-described compound having an alkyleneoxy group preferably has an aromatic ring group.

The aromatic ring group may be any of an aryl group and a heteroaryl group.

The aryl group may be any of a monocyclic group and a fused ring group, and is preferably a monocyclic group. The number of carbon atoms in the aryl group (the number of carbon atoms in the substituent as a whole, including a substituent that may be included in the aryl group) is preferably 6 to 50, more preferably 6 to 46, and still more preferably 6 to 38.

The aryl group may be unsubstituted or may have a substituent. Examples of the substituent that may be included in the aryl group include an alkyl group (preferably having 1 to 6 carbon atoms and more preferably having 1 to 4 carbon atoms), an alkenyl group (preferably having 2 to 6 carbon atoms and more preferably having 2 to 4 carbon atoms), an aryl group (preferably having 6 to 10 carbon atoms and more preferably having 6 carbon atoms), an alkoxy group (preferably having 1 to 6 carbon atoms and more preferably having 1 to 4 carbon atoms), and an aryloxy group (preferably having 6 to 10 carbon atoms and more preferably having 6 carbon atoms). These groups may be further substituted with an alkyl group, an aryl group, or the like. Specific examples of the substituent that may be included in the aryl group include a styryl group, a benzyl group, a cumyl group, a naphthyl group, a biphenyl group, and a phenoxy group.

The heteroaryl group is an aromatic heterocyclic group composed of carbon atoms and heteroatoms (such as an oxygen atom and a sulfur atom), and may be any of a monocyclic group and a fused ring group. The number of atoms constituting the aromatic heterocyclic ring is preferably 5 to 20 and more preferably 5 to 14. The number of heteroatoms in the atoms constituting the ring is preferably 1 to 3 and more preferably 1 or 2.

Examples of the heteroaryl group include a furyl group, a thienyl group, and a benzofuranyl group.

The heteroaryl group may be unsubstituted or may have a substituent. The substituent that may be included in the heteroaryl group may be as described in the substituent that may be included in the aryl group.

The aromatic ring group is preferably an aryl group.

The nonionic dispersing agent (N) is preferably a polyoxyalkylene-based dispersing agent. The polyoxyalkylene-based dispersing agent means a nonionic dispersing agent having the above-described alkyleneoxy group (monoalkyleneoxy group or polyalkyleneoxy group).

Examples of the polyoxyalkylene-based dispersing agent include polyoxyalkylene alkyl ether, polyoxyalkylene fatty acid ester, polyoxyalkylene aryl ether, polyoxyalkylene sorbitan fatty acid ester, and polyoxyalkylene fatty acid amide. It is preferable to contain at least one of these. It is more preferable to contain at least one of polyoxyalkylene alkyl ether, polyoxyalkylene fatty acid ester, polyoxyalkylene aryl ether, or polyoxyalkylene sorbitan fatty acid ester. It is still more preferable to contain polyoxyalkylene aryl ether.

The polyoxyalkylene group in these polyoxyalkylene-based dispersing agents may be as described in the alkyleneoxy group (monoalkyleneoxy group or polyalkyleneoxy group). The polyoxyalkylene group is, for example, an ethyleneoxy group (monoethyleneoxy group or polyethyleneoxy group), a propyleneoxy group (monopropyleneoxy group or polypropyleneoxy group), or a group having both of these groups (monoethyleneoxy-monopropyleneoxy group, polyethyleneoxy-polypropyleneoxy group, or the like).

The polyoxyalkylene alkyl ether may be any of a polyoxyalkylene monoalkyl ether and a polyoxyalkylene dialkyl ether, and is preferably a polyoxyalkylene monoalkyl ether. The number of carbon atoms of the alkyl group in the polyoxyalkylene alkyl ether is, for example, preferably 6 to 20 and more preferably 8 to 18. The alkyl group may be either of a linear group or a branched group, and is preferably a linear group. The polyoxyalkylene alkyl ether may have a structure derived from a polyhydric alcohol such as glycerin in an alkyl moiety thereof, and is, for example, polyoxyalkylene glyceryl ether.

The polyoxyalkylene fatty acid ester may be any of a polyoxyalkylene monofatty acid ester or a polyoxyalkylene difatty acid ester, and is preferably a polyoxyalkylene monofatty acid ester. The number of carbon atoms of the fatty acid group in the polyoxyalkylene fatty acid ester is, for example, preferably 6 to 20 and more preferably 8 to 18. The fatty acid group may be either of a linear group or a branched group, and is preferably a linear group.

The polyoxyalkylene sorbitan fatty acid ester may be any polyoxyalkylene sorbitan fatty acid ester in which at least one hydroxy group of four hydroxy groups in a sorbitan is bonded to a polyoxyalkylene group, and at least one fatty acid ester is formed by at least one hydroxy group of the four hydroxy groups in the sorbitan and/or the above-described polyoxyalkylene group and a fatty acid. The fatty acid group in the polyoxyalkylene sorbitan fatty acid ester is preferably as described in the fatty acid group in the polyoxyalkylene fatty acid ester.

The polyoxyalkylene fatty acid amide may be any of a polyoxyalkylene monofatty acid amide and a polyoxyalkylene difatty acid amide. The fatty acid group in the polyoxyalkylene fatty acid amide is preferably as described in the fatty acid group in the polyoxyalkylene fatty acid ester.

The polyoxyalkylene aryl ether may be any of a polyoxyalkylene monoaryl ether and a polyoxyalkylene diaryl ether, and is preferably a polyoxyalkylene monoaryl ether.

The aryl group in the polyoxyalkylene aryl ether is preferably as described in the above aryl group. The aryl group is, for example, preferably a phenyl group or a naphthyl group that may be substituted with a nonyl group, a phenyl group, a styryl group, a benzyl group, a cumyl group, a naphthyl group, a biphenyl group, or a phenoxy group. **In** a case where the phenyl group or the naphthyl group has a nonyl group, a phenyl group, a styryl group, a benzyl group, a cumyl group, a naphthyl group, a biphenyl group, or a phenoxy group as a substituent, the number of substituents is not particularly limited and can be set to, for example, 1 to 5.

Examples of the polyoxyalkylene aryl ether include styrylphenol compounds (polyoxyalkylene styrylphenyl ether) such as polyoxyethylene styrylphenyl ether, polyoxyethylene distyrylphenyl ether, polyoxyethylene tristyrylphenyl ether, and polyoxyethylene tetrastyrylphenyl ether; benzylphenol compounds such as polyoxyethylene benzylphenyl ether, polyoxyethylene dibenzylphenyl ether, and polyoxyethylene tribenzylphenyl ether; cumylphenol compounds such as polyoxyethylene cumylphenyl ether; nonylphenol compounds (polyoxyalkylene nonylphenyl ether) such as polyoxyethylene nonylphenyl ether; and polyoxyethylene naphthylphenyl ether, polyoxyethylene naphthyl ether, polyoxyethylene biphenyl ether, and polyoxyethylene phenoxyphenyl ether. **In** these examples, compounds in which the polyoxyethylene moiety is substituted with polyoxypropylene (for example, polyoxypropylene monostyrylphenyl ether) and compounds in which the polyoxyethylene moiety is substituted with polyoxyethylene-polyoxypropylene (for example, polyoxyethylene-polyoxypropylene monostyrylphenyl ether) are also preferably included.

The average repeating number of the polyoxyalkylene group in the polyoxyalkylene arylphenyl ether such as a polyoxyethylene group in the polyoxyethylene arylphenyl ether is preferably 1 to 30, more preferably 2 to 30, and still more preferably 15 to 30. When the repeating number is 1 or more, the compatibility with an aqueous solvent or the like tends to be good. When the repeating number is 30 or less, the viscosity tends not to be excessively high.

Among the polyoxyalkylene arylphenyl ethers, at least one of polyoxyalkylene styrylphenyl ether such as polyoxyethylene styrylphenyl ether, polyoxypropylene styrylphenyl ether, or polyoxyethylene-polyoxypropylene styrylphenyl ether and polyoxyalkylene nonylphenyl ether such as polyoxyethylene nonylphenyl ether, polyoxypropylene nonylphenyl ether, or polyoxyethylene polyoxypropylene nonylphenyl ether is preferably included. The number of styryl groups or nonyl groups substituted on the phenyl group of the phenyl ether is not particularly limited, and may be 1 to 5.

The number-average molecular weight (Mn) of the nonionic dispersing agent (N) is preferably 100 or more, more preferably 200 or more, and still more preferably 300 or more. The upper limit value is preferably 4,000 or less, more preferably 2,000 or less, still more preferably 1,500 or less, and particularly preferably 1,000 or less. That is, the number-average molecular weight (Mn) of the nonionic dispersing agent (N) is preferably 100 to 4,000, more preferably 100 to 2,000, still more preferably 200 to 1,500, and particularly preferably 300 to 1,000.

In the present invention, the number-average molecular weight is a value measured by a method described in Examples below, and is also simply referred to as a molecular weight in the present invention.

The hydrophile-lipophile balance (HLB) value of the nonionic dispersing agent (N) is usually 2 to 18, preferably 4 to 16, more preferably 6 to 15, and particularly preferably 9 to 14.

In the present invention, the HLB value of a nonionic surfactant is a value actually measured by an emulsification method according to the method described in "Handbook-Cosmetic/Preparation Raw Materials-Revised Edition", published by Nikko Chemicals Co., Ltd. on February 1, 1977, p. 854 to 855.

It is presumed that the nonionic dispersing agent (N) can improve the adhesiveness between a coating layer and a to-be-coated member (substrate) in the to-be-coated member whose surface has been subjected to a coating treatment using the composition according to the present invention, which can exhibit good bending durability.

The adhesiveness between the substrate and the coating layer is mainly controlled by a binder, and it is important to increase the bonding strength between the binder (thermosetting resin such as a polyamideimide resin) and SUS (substrate). The present inventor has found that, for the principle of expressing the bonding property, when a coating composition using a dispersing agent of a solid lubricant in the related art is used, a charge involved in an ionic bond between the binder and the substrate is neutralized by an ionic group included in the dispersing agent, and thus the adhesiveness between the binder and the substrate is lowered. Therefore, it is considered that, by using the nonionic dispersing agent instead of a dispersing agent having an acid value and/or an amine value, a decrease in bonding property between the binder and the substrate can be suppressed, which can exhibit good bending durability.

In particular, from the viewpoint of achieving better bending durability, it is preferable to use a dispersing agent having an aromatic ring group as the nonionic dispersing agent (N) because the adhesiveness with the solid lubricant, particularly graphite made of carbon, tends to be increased.

It is also presumed that the nonionic dispersing agent (N) can suppress deterioration of the coating layer due to a sterilization treatment in the to-be-coated member whose surface has been subjected to a coating treatment using the composition according to the present invention, which can exhibit good sterilization resistance.

In a case where the coating layer is subjected to a low-temperature gas sterilization treatment having a strong chemical force, chemical bonds of organic components are mainly broken, and a binder component is deteriorated. As a result, the coating layer is deteriorated. The present inventor has found that the deterioration of the coating layer due to a H₂O₂ gas is likely to occur particularly in a component having a low molecular weight, and the deterioration of the binder component occurs due to the deterioration of the dispersing agent even though the amount added is small. The nonionic dispersing agent (N) used in the present invention is a nonionic dispersing agent not having an acid value and/or an amine value, and is a compound constituted by limited specific elements of a carbon element, a hydrogen element, and at least one of an oxygen element or a nitrogen element. Therefore, it is presumed that the deterioration due to the H₂O₂ gas is less likely to occur, and the sterilization resistance of the entire coating layer is improved.

When the nonionic dispersing agent (N) is the above-described compound having an alkyleneoxy group (a monoalkyleneoxy group or a polyalkyleneoxy group), particularly when a dispersing agent having a propyleneoxy structure is used instead of the dispersing agent having an ethyleneoxy structure as an alkyleneoxy structure constituting the alkyleneoxy group, the H₂O₂ gas resistance is further improved. This is presumably because the presence of a -CH₃ group in the side chain suppresses the cleavage of a molecular chain constituting the main chain caused by strong H₂O₂ gas-derived active radicals.

Similarly, since the atmosphere becomes acidic in a case of being exposed to the H₂O₂ gas, an ester bond and an amide bond are likely to be cleaved, and a dispersing agent having an ether structure that is resistant to a pH change is preferable. Furthermore, a compound having an aromatic ring group is more preferable than an aliphatic compound having poor oxidation stability in terms of good sterilization resistance.

When the nonionic dispersing agent (N) has a polymer structure, the polymer structure is not particularly limited. The polymer structure is, for example, preferably a random polymer-type copolymer, a block polymer-type copolymer, or a graft polymer-type copolymer.

For example, when the nonionic dispersing agent (N) is a polymer, a good nonionic dispersing agent (N) can be obtained by a typical method by copolymerizing the above-described monomer component having a nonionic water-soluble group with another monomer component having lower hydrophilicity (or also referred to as higher hydrophobicity) than the monomer component.

The nonionic dispersing agent (N) can also be commercially available. The commercially available nonionic dispersing agent (N) is, for example, as follows.

Examples of a commercially available product of the polyoxyethylene styrylphenyl ether include, in terms of trade names, TAKESURF D-6112, TAKESURF D-6115, TAKESURF D-6120, TAKESURF D-6131, TAKESURF D-6512, TAKESURF D-6413, DTD-51, and TAKESURF D-6320 (all of which are manufactured by TAKEMOTO OIL & FAT Co., Ltd.); TS-1500, TS-2000, TS-2600, and SM-174N (all of which are manufactured by TOHO Chemical Industry Co., Ltd.); and EMULGEN A-60, EMULGEN A-90, and EMULGEN A-500 (all of which are manufactured by Kao Corporation).

Examples of a commercially available product of the polyoxyethylene naphthyl ether include, in terms of trade names, NOIGEN EN series (manufactured by Daiichi Kogyo Seiyaku Co., Ltd.) and TAKESURF D-7240 (manufactured by TAKEMOTO OIL & FAT Co., Ltd.).

In the composition according to the present invention, the content of the nonionic dispersing agent (N) is preferably 0.1% to 20% by mass, more preferably 0.1% to 15% by mass, still more preferably 0.2% to 10% by mass, still more preferably 0.2% to 8% by mass, still more preferably 0.2% to 5% by mass, still more preferably 0.2% to 3% by mass, and particularly preferably 0.3% to 2% by mass.

### Thermosetting resin

The composition according to the present invention includes a thermosetting resin. In the to-be-coated member treated with the composition according to the present invention, the thermosetting resin functions as a binder, and the bending durability and the sterilization resistance of the to-be-coated member can be enhanced to a high degree.

The thermosetting resin is not particularly limited. Examples of the thermosetting resin include a polyamideimide resin, a polyimide resin (including a polyimide resin present in a composition as a polyamic acid), an epoxy resin, a phenol resin, a melamine resin, and a urea resin. Among them, the thermosetting resin preferably includes a polyamideimide resin, and more preferably, the thermosetting resin is a polyamideimide resin.

The above-described polyamideimide resin preferably includes, as a constituent component of the polyamideimide, a structural unit represented by Formula (1) below.

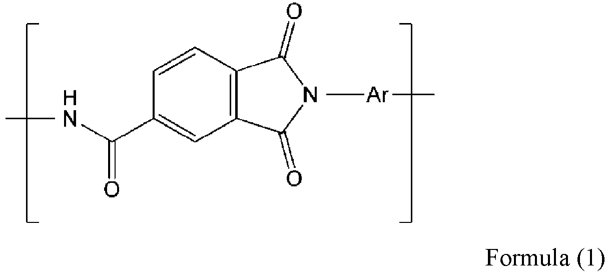

**In** the formula, Ar represents a divalent group having an aromatic ring.

Ar is preferably a phenylene group, a diphenylenemethane group (-phenylene-CH₂-phenylene-), a diphenylenepropane group (-phenylene-C₃H₆-phenylene-), a diphenylene ether group (-phenylene-O-phenylene-), a diphenylenesulfone group (-phenylene-SO₂-phenylene-), a diphenyleneamine group (-phenylene-NH-phenylene-), a biphenyldiyl group (-phenylene-phenylene-), or a naphthalenediyl group, and more preferably a phenylene group, a diphenylenemethane group, a diphenylenepropane group, a diphenylene ether group, or a diphenylene amine group.

**In** the polyamideimide, the content of the structural unit represented by the Formula (1) is preferably 50% by mass or more, more preferably 70% by mass or more, still more preferably 80% by mass or more, and particularly preferably 90% by mass or more. The polyamideimide is also preferably in a form constituted by the structural unit represented by the Formula (1).

The polyamideimide preferably has a weight-average molecular weight of 10,000 to 100,000, more preferably 20,000 to 80,000, and still more preferably 25,000 to 60,000.

**In** the present invention, the weight-average molecular weight is a value measured by a method described in Examples later.

**In** the composition according to the present invention, the content of the thermosetting resin is preferably 0.5% to 20% by mass, more preferably 1% to 15% by mass, still more preferably 2% to 10% by mass, and particularly preferably 3% to 8% by mass.

**In** the composition according to the present invention, the content of the nonionic dispersing agent (N) is preferably 0.5 to 50 parts by mass, more preferably 1 to 30 parts by mass, still more preferably 1 to 20 parts by mass, and particularly preferably 2 to 15 parts by mass with respect to 100 parts by mass of the content of the solid lubricant.

**In** the composition according to the present invention, the content of the thermosetting resin is preferably 1 to 50 parts by mass, more preferably 5 to 70 parts by mass, still more preferably 10 to 60 parts by mass, and particularly preferably 12 to 50 parts by mass with respect to 100 parts by mass of the content of the solid lubricant.

**In** the composition according to the present invention, the content of the nonionic dispersing agent (N) is preferably 2 to 100 parts by mass, more preferably 3 to 90 parts by mass, still more preferably 4 to 80 parts by mass, still more preferably 5 to 70 parts by mass, still more preferably 5 to 60 parts by mass, still more preferably 6 to 50 parts by mass, and particularly preferably 6 to 40 parts by mass with respect to 100 parts by mass of the thermosetting resin.

### Solvent

The composition according to the present invention includes a solvent as a dispersion medium for the solid lubricant. The solvent is not particularly limited as long as it functions as a dispersion medium. The solvent used is preferably an organic solvent.

Examples of the solvent that may be used in the composition according to the present invention include aprotic polar solvents such as N-methyl-2-pyrrolidone (NMP), N,N-dimethylacetamide, N,N-dimethylformamide, hexamethylphosphoric triamide, γ-butyrolactone, and 1,3-dimethyl-2-imidazolidinone, and xylene.

The composition according to the present invention preferably includes, as a solvent, one or two or more solvents in a combined manner that include at least one of the above-described aprotic polar solvents and that may be used in the composition according to the present invention.

**In** the composition according to the present invention, the proportion of all the aprotic polar solvents in the entire solvent can be, for example, set to 40% by mass or more, preferably 50% by mass or more, and more preferably 60% by mass or more. It is also preferable that all of the solvents are aprotic polar solvents.

**In** the composition according to the present invention, the content of the solvent is not particularly limited as long as the solvent can function as a dispersion medium for the solid lubricant and can form the composition together with the thermosetting resin. For example, the content of the solvent in the composition according to the present invention can be set to 10% to 90% by mass and is preferably 20% to 85% by mass, more preferably 20% to 80% by mass, and still more preferably 40% to 80% by mass.

The composition according to the present invention can appropriately include various general additives, in addition to the above-described components, without impairing the effects of the present invention. Examples of such an additive include a plasticizer, a colorant, an anti-foaming agent, a surfactant, and a dispersing agent.

### Preparation of coating composition for endoscope

The composition according to the present invention can be obtained by uniformly mixing each of the above-described components using a homogenizer or the like.

In the present invention, the "composition" encompasses not only a form in which the components are uniformly present in the composition, but also a form in which some of the components are present in a state of being unevenly distributed without impairing the effects of the present invention.

The composition according to the present invention can be suitably used as a coating agent for imparting sliding properties (lubricity) to various members (members for endoscopes) used in endoscopes. For example, the composition can be applied to a surface of a wire inserted into the endoscope insertion part in order to bend a bending part of the endoscope insertion part in a desired direction. This can effectively reduce the friction between the wire and the wire guide, and can also improve the bending durability and sterilization resistance of the wire. The composition can also be applied to a resin coating layer surface of a flexible tube for an endoscope.

### Lubricating member for endoscope

The lubricating member for an endoscope according to the present invention (hereinafter also referred to as a "lubricating member according to the present invention") is a member for an endoscope that has been subjected to a coating treatment with the composition according to the present invention. A method of performing the coating treatment with the composition according to the present invention is not particularly limited. For example, the composition according to the present invention is applied to a surface of a to-be-coated member, and then dried to remove the solvent, thereby coating the surface of the to-be-coated member with a solid lubricant (forming a coating layer on the surface of the to-be-coated member). The coating can be performed by a spray method, a dipping method, a coating method with a brush or a roller, a bar coating method, or the like. After the composition according to the present invention is applied onto the surface of the to-be-coated member, heating is performed to cure the thermosetting resin. Therefore, in the lubricating member according to the present invention, the thermosetting resin in the composition according to the present invention is present in a thermoset state. The heating may be performed at the same time as that of the removal of the solvent through the drying and/or after the removal of the solvent. The conditions such as a heating temperature and a heating time in the heating can be appropriately adjusted according to the type of the thermosetting resin as long as the thermosetting resin can be cured.

The film thickness of the coating layer is not particularly limited as long as the effects of the present invention are exhibited, and can be, for example, set to 1 to 500 µm, preferably 5 to 100 µm.In the present invention, the film thickness of the coating layer is a value measured by a method described in Examples later.

The lubricating member according to the present invention is preferably a lubricating wire for an endoscope. As described above, the lubricating wire for an endoscope can be obtained by applying the composition according to the present invention to the surface of the wire to be inserted into the endoscope insertion part.

### Flexible tube for endoscope and Endoscope

The endoscope of the present invention includes a flexible tube for an endoscope according to the present invention.

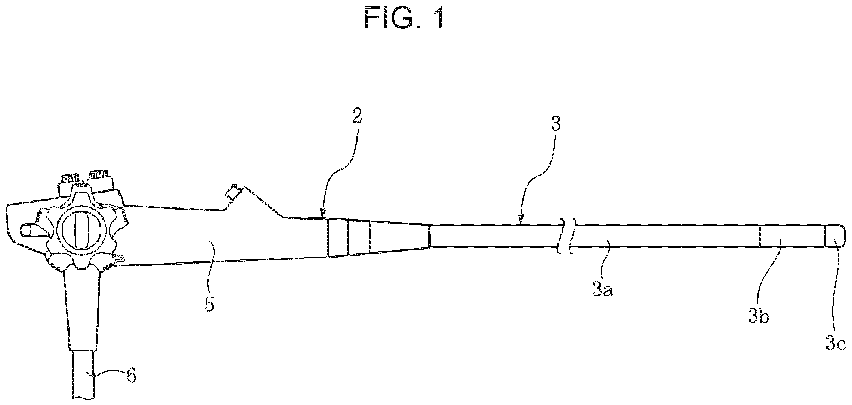

A preferred form of the endoscope of the present invention will be described with reference to an electronic endoscope. In the electronic endoscope, a flexible tube for an endoscope is incorporated (hereinafter, the flexible tube for an endoscope may be simply referred to as a "flexible tube"), and the electronic endoscope is used as a medical device for, for example, observing a body cavity by inserting the flexible tube into the body cavity. In an example illustrated in Fig. 1, an electronic endoscope 2 includes an insertion part 3 to be inserted into a body cavity, a main body operating unit 5 that is consecutively provided at a proximal end portion of the insertion part 3, and a universal cord 6 connected to a processor device or a light source device. The insertion part 3 is constituted by a flexible tube 3a that is consecutively provided to the main body operating unit 5, a bending part 3b (angle part 3b) that is consecutively provided to the flexible tube 3a, and a tip part 3c that is consecutively provided to the tip of the bending part 3b and that includes a built-in imaging device (not illustrated) for imaging an inside of the body cavity. The flexible tube 3a that occupies most of the length of the insertion part 3 has flexibility over substantially the entire length thereof, and particularly a portion to be inserted into an inside of the body cavity or the like has a structure having more flexibility. As described above, the bending part 3b can be bent in a desired direction by pulling the wire inserted into the insertion part 3 and fixed to the inner wall of the bending part 3b by the wire guide. As an example of the form of bending using a wire, for example, Fig. 1 of JP2009-180A can be referred to.

The flexible tube for an endoscope according to the present invention includes the lubricating member according to the present invention. As described above, examples of the lubricating member included in the flexible tube for an endoscope according to the present invention include a wire and a resin coating layer.

### Examples

Hereinafter, the present invention will be described in more detail with reference to Examples. However, it is to be understood that the present invention is not limited by these Examples.

### Example 1

### <Preparation Example 1-1> Preparation of coating composition (C-1) for endoscope

In a container of a small homogenizer (manufactured by NIHONSEIKI KAISHA LTD., trade name: EXCEL AUTO HOMOGENIZER ED-7, impeller diameter: 45 mmφ), 20.0 g (5.2 g in terms of solid content, 14.8 g of N-methyl-2-pyrrolidone) of a polyamideimide resin solution (manufactured by Resonac Co., Ltd. (formerly: Showa Denko Materials Co., Ltd.), trade name: HPC-6000-26, thermosetting resin, weight-average molecular weight: 40,000, solid content: 26% by mass), 37.2 g of N-methyl-2-pyrrolidone (manufactured by FUJIFILM Wako Pure Chemical Corporation, first-grade reagent), 22.3 g of p-xylene (manufactured by FUJIFILM Wako Pure Chemical Corporation, first-grade reagent), and 0.5 g of a nonionic dispersing agent (polyoxyethylene octyl ether, manufactured by TAKEMOTO OIL & FAT Co., Ltd., trade name: TAKESURF D-1004, HLB value: 11.5) were put, and mixing was performed by stirring at 1,000 rotations per minute (rpm) for 5 minutes.

Subsequently, 20.0 g of a graphite powder (manufactured by Fuji Graphite Works Co., Ltd., trade name: MF-10N, particle diameter: 10 µm) was added thereto and mixed by stirring at 3000 rpm for 30 minutes to obtain a coating composition of Example 1.

### <Preparation Example 1-2> Preparation of lubricating member for endoscope - 1

The entire surface of a stainless wire (made of SUS303, 0.5 mmφ) was spray-coated with the coating composition of Example 1 using a hand gun. Next, the polyamideimide resin was cured by performing heating and drying in an oven at 230°C for 20 minutes, thereby producing a lubricating wire for an endoscope of Example 1. In this lubricating wire for an endoscope, the coating layer had a film thickness of 19 µm. A film thickness was measured at 5 points at random using a micrometer, and the average value of the obtained 5 measured values was defined as the film thickness.

### Examples 2 to 41 and Comparative Examples 1 to 3

Coating compositions of Examples 2 to 41 and Comparative Examples 1 to 3 were obtained in the same manner as in Preparation Example 1-1, except that the raw materials used were changed as shown in Tables below.

Next, lubricating wires for an endoscope of Examples 2 to 41 and Comparative Examples 1 to 3 were obtained in the same manner as in Preparation Example 1-2 using the obtained coating compositions.

In each of the lubricating wires for an endoscope of Examples 2 to 41 and Comparative Examples 1 to 3, the film thicknesses of the coating layers measured in the same manner as in Example 1 were 10 to 30 µm.

The obtained coating compositions and the obtained lubricating wires for an endoscope were evaluated as follows. Tables below collectively show the results.

### [Test Example 1] Evaluation of liquid dispersion stability

The viscosity (I) of the coating composition prepared in each of Preparation Examples immediately after the preparation was measured by the following measuring method. Next, each coating composition was allowed to stand at 5°C for 30 days. Thereafter, the coating composition was stirred at 100 rpm for 30 minutes, and the viscosity (II) was measured again by the following measuring method.

### Method for measuring viscosity

Using RheoStress RS6000 (trade name) manufactured by HAAKE, the dynamic complex viscosity at 27°C and 10 Hz was measured and defined as a viscosity of the coating composition. The unit of the viscosity is Pa·s.

A rate of change in viscosity (%) was calculated by the following formula using the obtained viscosities (I) and (II), and the rate of change in viscosity was applied to the following evaluation criteria to evaluate the dispersion stability. Rate of change in viscosity (%) = 100 × [viscosity (II) - viscosity (I)]/viscosity (I) Evaluation criteria of liquid dispersion stability
A: Rate of change in viscosity was less than 10%, and deposition of solid matter on the bottom surface of the container was not present.
B: Rate of change in viscosity was 10% or more and less than 100%, and deposition of solid matter on the bottom surface of the container was not present.
C: Rate of change in viscosity was 100% or more, and deposition of solid matter on the bottom surface of the container was not present.
D: Rate of change in viscosity was 100% or more, and deposition of solid matter on the bottom surface of the container was present.

The phrase "deposition of solid matter on the bottom surface of the container was present" means that when the container was allowed to stand at 5°C for 30 days and then the coating composition was taken out from the container, a viscous liquid having a viscosity of 1000 Pa·s or more or a solid remained on the bottom surface of the container.

### [Test Example 2] Evaluation of bending durability

The lubricating wire for an endoscope produced in each of Preparation Examples was wound around a pulley having a diameter of 20 mm for one complete turn. A bending test was performed in which the wire was slid back and forth 100 times in the length direction while applying a tension of 10 gf (1 kgf = 9.8 N), and the peeling state of the coating layer was observed. When the peeling of the coating layer did not occur, the diameter of the pulley was decreased and the bending test was performed in the same manner as that of the case of using the above-described pulley having a diameter of 20 mm. The bending test was performed by decreasing the diameter of the pulley until peeling occurred. The diameter of the pulley used when peeling occurred was applied to the following criteria, and the bending durability was evaluated.

### Evaluation criteria of bending durability

AA: less than 8 mm
A: 8 mm or more and less than 12 mm
B: 12 mm or more and less than 16 mm
C: 16 mm or more and less than 20 mm
D: 20 mm

### [Test Example 3] Evaluation of sterilization resistance against hydrogen peroxide gas

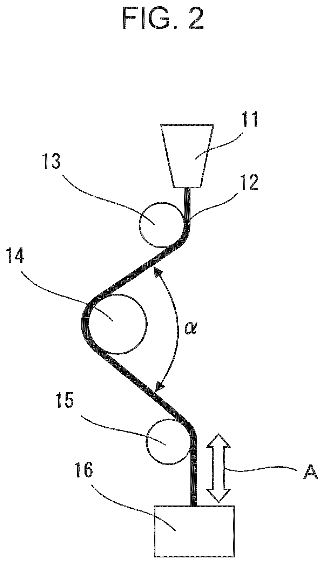

The lubricating wire for an endoscope produced as described above was subjected to a sliding property test using a test instrument illustrated in Fig. 2. As illustrated in Fig. 2, a lubricating wire (12) for an endoscope obtained in each of Preparation Examples was set on a SUS rod (14) of φ5 mm and pulleys (13, 15) so as to have an angle (α) of 100 degrees. A load of 10 N was applied using a weight (16), and this state was defined as an initial state of the test.

Next, the weight was pulled downward at a stroke (A) of 10 mm and a rate of 100 mm/min, and then returned to the original state. This operation was set as one reciprocating motion, and the maximum stress measured by a force gauge (11) during this one reciprocating motion was recorded.

Next, the wire that had been measured for the maximum stress was subjected to a low-temperature plasma sterilization treatment repeatedly 100 times on an advance course of a hydrogen peroxide gas sterilizer ("STERRAD" (registered trademark) NX manufactured by ASP). Thereafter, the maximum stress was measured by performing the same sliding property test as described above. When the maximum stress before the sterilization treatment was defined as "PSB(2)" and the maximum stress after the sterilization treatment was defined as "PSA(2)", the proportion of "PSA(2)" to "PSB(2)", {(PSA(2)/PSB(2)) × 100 (%)}, was determined and applied to the following criteria to evaluate the sterilization resistance against hydrogen peroxide gas.

The maximum stress PSB(2) of each of the lubricating wires for an endoscope of Examples 1 to 41 before the sterilization treatment was less than 20 N, which exhibited lubricity that was practically not a problem.

### Evaluation criteria

AA: 80% or more
A: 60% or more and less than 80%
B: 40% or more and less than 60%
C: 20% or more and less than 40%
D: less than 20%

### Measurement of molecular weight

The number-average molecular weights (Mn) of the dispersing agent and the binder and the weight-average molecular weight (Mw) of the binder were measured as molecular weights in terms of polystyrene by gel permeation chromatography (GPC). At this time, a GPC instrument HLC-8220 (trade name, manufactured by Tosoh Corporation) was used, tetrahydrofuran (THF) was used as an eluant, G3000HXL + G2000HXL (trade name) were used as columns, and a refractive index (RI) detector was used under the measurement conditions of 23°C and a flow rate of 1 mL/min.

### Table 1

**Table 1**

| | | Example 1 | Example 2 | Example 3 | Example 4 | Example 5 | Example 6 | Example 7 | Example 8 | Example 9 | Example 10 |
|---|---|---|---|---|---|---|---|---|---|---|---|
| Binder (B) | Type | (B-1) | (B-1) | (B-1) | (B-1) | (B-1) | (B-1) | (B-1) | (B-1) | (B-1) | (B-1) |
| | Blending amount | 5.2 | 5.2 | 5.2 | 5.2 | 5.2 | 5.2 | 5.2 | 5.2 | 5.2 | 5.2 |
| Inorganic solid lubricant (S) | Type | (S-1) | (S-1) | (S-1) | (S-1) | (S-1) | (S-1) | (S-1) | (S-1) | (S-1) | (S-1) |
| | | Graphite | Graphite | Graphite | Graphite | Graphite | Graphite | Graphite | Graphite | Graphite | Graphite |
| | Particle diameter [µm] | 10 | 10 | 10 | 10 | 10 | 10 | 10 | 10 | 10 | 10 |
| | Blending amount | 20 | 20 | 20 | 20 | 20 | 20 | 20 | 20 | 20 | 20 |
| Organic solid lubricant (G) | Type | | | | | | | | | | |
| | | | | | | | | | | | |
| | Particle diameter [µm] | | | | | | | | | | |
| | Blending amount | | | | | | | | | | |
| Dispersing agent (D) | Type | (D-1) | (D-2) | (D-3) | (D-4) | (D-5) | (D-6) | (D-7) | (D-8) | (D-9) | (D-10) |
| | Blending amount | 0.5 | 0.5 | 0.5 | 0.5 | 0.5 | 0.5 | 0.5 | 0.5 | 0.5 | 0.5 |
| Solvent | Type | NMP | NMP | NMP | NMP | NMP | NMP | NMP | NMP | NMP | NMP |
| | Blending amount | 52.0 | 52.0 | 52.0 | 52.0 | 52.0 | 52.0 | 52.0 | 52.0 | 52.0 | 52.0 |
| | Type | PX | PX | PX | PX | PX | PX | PX | PX | PX | PX |
| | Blending amount | 22.3 | 22.3 | 22.3 | 22.3 | 22.3 | 22.3 | 22.3 | 22.3 | 22.3 | 22.3 |
| Liquid dispersion stability | | A | A | B | c | A | A | A | A | A | A |
| Bending durability | | C | B | B | B | B | B | B | A | A | A |
| Sterilization resistance against hydrogen peroxide gas | | B | B | A | A | AA | A | B | A | AA | A |

### Table 2

**Table 2**

| | | Example 11 | Example 12 | Example 13 | Example 14 | Example 15 | Example 16 | Example 17 | Example 18 | Example 19 | Example 20 |
|---|---|---|---|---|---|---|---|---|---|---|---|
| Binder (B) | Type | (B-1) | (B-1) | (B-1) | (B-1) | (B-1) | (B-1) | (B-1) | (B-1) | (B-1) | (B-1) |
| | Blending amount | 5.2 | 5.2 | 5.2 | 5.2 | 5.2 | 5.2 | 5.2 | 5.2 | 5.2 | 5.2 |
| Inorganic solid lubricant (S) | Type | (S-1) | (S-1) | (S-1) | (S-1) | (S-1) | (S-2) | (S-3) | (S-4) | (S-5) | (S-6) |
| | | Graphite | Graphite | Graphite | Graphite | Graphite | Graphite | Graphite | Graphite | Graphite | Graphite |
| | Particle diameter [µm] | 10 | 10 | 10 | 10 | 10 | 1.0 | 3.2 | 20 | 45 | 63 |
| | Blending amount | 20 | 20 | 20 | 20 | 20 | 20 | 20 | 20 | 20 | 20 |
| Organic solid lubricant (G) | Type | | | | | | | | | | |
| | | | | | | | | | | | |
| | Particle diameter [µm] | | | | | | | | | | |
| | Blending amount | | | | | | | | | | |
| Dispersing agent (D) | Type | (D-11) | (D-12) | (D-13) | (D-14) | (D-15) | (D-9) | (D-9) | (D-9) | (D-9) | (D-9) |
| | Blending amount | 0.5 | 0.5 | 0.5 | 0.5 | 0.5 | 0.5 | 0.5 | 0.5 | 0.5 | 0.5 |
| Solvent | Type | NMP | NMP | NMP | NMP | NMP | NMP | NMP | NMP | NMP | NMP |
| | Blending amount | 52.0 | 52.0 | 52.0 | 52.0 | 52.0 | 52.0 | 52.0 | 52.0 | 52.0 | 52.0 |
| | Type | PX | PX | PX | PX | PX | PX | PX | PX | PX | PX |
| | Blending amount | 22.3 | 22.3 | 22.3 | 22.3 | 22.3 | 22.3 | 22.3 | 22.3 | 22.3 | 22.3 |
| Liquid dispersion stability | | A | A | A | A | A | B | B | A | A | A |
| Bending durability | | A | A | B | B | B | A | A | A | B | c |
| Sterilization resistance against hydrogen peroxide gas | | AA | A | B | B | B | AA | AA | AA | A | A |

### Table 3

**Table 3**

| | | Example 21 | Example 22 | Example 23 | Example 24 | Example 25 | Example 26 | Example 27 | Example 28 | Example 29 | Example 30 |
|---|---|---|---|---|---|---|---|---|---|---|---|
| Binder (B) | Type | (B-1) | (B-1) | (B-1) | (B-1) | (B-1) | (B-1) | (B-1) | (B-1) | (B-1) | (B-1) |
| | Blending amount | 5.2 | 5.2 | 5.2 | 5.2 | 5.2 | 5.2 | 5.2 | 5.2 | 5.2 | 5.2 |
| Inorganic solid lubricant (S) | Type | (S-7) | (S-8) | (S-9) | (S-10) | | | | (S-1) | (S-1) | (S-1) |
| | | F-graphite | Graphene | CNT | BN | | | | Graphite | Graphite | Graphite |
| | Particle diameter [µm] | 5.0 | 4.5 | 5.0 | 4.0 | | | | 10 | 10 | 10 |
| | Blending amount | 20 | 20 | 20 | 20 | | | | 10 | 10 | 10 |
| Organic solid lubricant (G) | Type | | | | | (G-1) | (G-2) | (G-3) | (G-1) | (G-2) | (G-3) |
| | | | | | | PTFE | Melamine | Guanamine | PTFE | Melamine | Guanamine |
| | Particle diameter [µm] | | | | | 3.0 | 2.0 | 9 | 3.0 | 2.0 | 9 |
| | Blending amount | | | | | 20 | 20 | 20 | 10 | 10 | 10 |
| Dispersing agent (D) | Type | (D-9) | (D-9) | (D-9) | (D-9) | (D-9) | (D-9) | (D-9) | (D-9) | (D-9) | (D-9) |
| | Blending amount | 0.5 | 0.5 | 0.5 | 0.5 | 0.5 | 0.5 | 0.5 | 0.5 | 0.5 | 0.5 |
| Solvent | Type | NMP | NMP | NMP | NMP | NMP | NMP | NMP | NMP | NMP | NMP |
| | Blending amount | 52.0 | 52.0 | 52.0 | 52.0 | 52.0 | 52.0 | 52.0 | 52.0 | 52.0 | 52.0 |
| | Type | PX | PX | PX | PX | PX | PX | PX | PX | PX | PX |
| | Blending amount | 22.3 | 22.3 | 22.3 | 22.3 | 22.3 | 22.3 | 22.3 | 22.3 | 22.3 | 22.3 |
| Liquid dispersion stability | | A | A | A | A | A | A | A | A | A | A |
| Bending durability | | A | A | A | A | c | B | B | AA | AA | AA |
| Sterilization resistance against hydrogen peroxide gas | | AA | AA | AA | AA | A | B | B | AA | A | A |

### Table 4

**Table 4**

| | | Example 31 | Example 32 | Example 33 | Example 34 | Example 35 | Example 36 | Example 37 | Example 38 | Example 39 | Example 40 |
|---|---|---|---|---|---|---|---|---|---|---|---|
| Binder (B) | Type | (B-2) | (B-1) | (B-1) | (B-1) | (B-1) | (B-1) | (B-1) | (B-1) | (B-1) | (B-1) |
| | Blending amount | 5.2 | 3.5 | 7 | 5.2 | 5.2 | 5.2 | 5.2 | 5.2 | 5.2 | 5.2 |
| Inorganic solid lubricant (S) | Type | (S-1) | (S-1) | (S-1) | (S-1) | (S-1) | (S-1) | (S-1) | (S-1) | (S-1) | (S-1) |
| | | Graphite | Graphite | Graphite | Graphite | Graphite | Graphite | Graphite | Graphite | Graphite | Graphite |
| | Particle diameter [µm] | 10 | 10 | 10 | 10 | 10 | 10 | 10 | 10 | 10 | 10 |
| | Blending amount | 10 | 10 | 10 | 15 | 5 | 5 | 5 | 20 | 10 | 10 |
| Organic solid lubricant (G) | Type | (G-1) | (G-1) | (G-1) | (G-1) | (G-1) | (G-1) | (G-1) | (G-1) | (G-1) | (G-1) |
| | | PTFE | PTFE | PTFE | PTFE | PTFE | PTFE | PTFE | PTFE | PTFE | PTFE |
| | Particle diameter [µm] | 3.0 | 3.0 | 3.0 | 3.0 | 3.0 | 3.0 | 3.0 | 3.0 | 3.0 | 3.0 |
| | Blending amount | 10 | 10 | 10 | 5 | 15 | 5 | 10 | 10 | 10 | 10 |
| Dispersing agent (D) | Type | (D-9) | (D-9) | (D-9) | (D-9) | (D-9) | (D-9) | (D-9) | (D-9) | (D-9) | (D-9) |
| | Blending amount | 0.5 | 0.5 | 0.5 | 0.5 | 0.5 | 0.5 | 0.5 | 0.5 | 0.2 | 1 |
| Solvent | Type | NMP | NMP | NMP | NMP | NMP | NMP | NMP | NMP | NMP | NMP |
| | Blending amount | 52.0 | 53.7 | 50.2 | 52.0 | 52.0 | 59.0 | 55.5 | 45.0 | 52.0 | 52.0 |
| | Type | PX | PX | PX | PX | PX | PX | PX | PX | PX | PX |
| | Blending amount | 22.3 | 22.3 | 22.3 | 22.3 | 22.3 | 25.3 | 23.8 | 19.3 | 22.6 | 21.8 |
| Liquid dispersion stability | | A | A | A | A | A | A | A | A | c | A |
| Bending durability | | B | AA | AA | A | A | AA | AA | A | A | AA |
| Sterilization resistance against hydrogen peroxide gas | | A | AA | AA | AA | A | A | A | AA | A | AA |

### Table 5

**Table 5**

| | | Example 41 | | Comparative Example 1 | Comparative Example 2 | Comparative Example 3 |
|---|---|---|---|---|---|---|
| Binder (B) | Type | (B-1) | | (B-1) | (B-1) | (B-3) |
| | Blending amount | 5.2 | | 5.2 | 5.2 | 5.2 |
| Inorganic solid lubricant (S) | Type | (S-1) | | (S-1) | (S-1) | (S-1) |
| | | Graphite | | Graphite | Graphite | Graphite |
| | Particle diameter [µm] | 10 | | 10 | 10 | 10 |
| | Blending amount | 10 | | 10 | 10 | 10 |
| Organic solid lubricant (G) | Type | (G-1) | | (G-1) | (G-1) | (G-1) |
| | | PTFE | | PTFE | PTFE | PTFE |
| | Particle diameter [µm] | 3.0 | | 3.0 | 3.0 | 3.0 |
| | Blending amount | 10 | | 10 | 10 | 10 |
| Dispersing agent (D) | Type | (D-9) | | - | (D-16) | (D-9) |
| | Blending amount | 1.5 | | | 0.5 | 0.5 |
| Solvent | Type | NMP | | NMP | NMP | NMP |
| | Blending amount | 52.0 | | 52.0 | 52.0 | 52.0 |
| | Type | PX | | PX | PX | PX |
| | Blending amount | 21.3 | | 22.8 | 22.3 | 22.3 |
| Liquid dispersion stability | | A | | D | A | A |
| Bending durability | | AA | | D | D | D |
| Sterilization resistance against hydrogen peroxide gas | | A | | D | C | D |

In Tables above, the blending amount of each raw material is in units of parts by mass relative to 100 parts by mass of the coating composition. The blending amount other than that of the solvent, that is, each of the blending amounts of the binder, the solid lubricant, and the dispersing agent is a blending amount (solid content) excluding the solvent included in the raw material. The solvent included in the raw material is shown in Tables as a blending amount of the solvent.

A blank and "-" in Tables mean that the corresponding raw material is not included.

Each of the raw materials described in Tables above will be described below.

### Binder (B)

(B-1): Polyamideimide resin (manufactured by Resonac Co., Ltd. (formerly: Showa Denko Materials Co., Ltd.), trade name: "HPC-6000-26", weight-average molecular weight: 40,000, thermosetting resin in which the structural unit Ar in Formula (1) is a diphenylmethane structure, solid content: 26% by mass)
(B-2): Polyimide resin (manufactured by UNITIKA Ltd., trade name: "U-IMIDE AR", the product is polyamic acid and becomes a polyimide resin by a thermal curing reaction, solid content: 18% by mass)
(B-3): Amorphous polyester resin (manufactured by Toyobo Co., Ltd., trade name: "VYLON 200", number-average molecular weight: 17,000, solid content: 100% by mass)

### Dispersing agent (D)

(D-1): Polyoxyethylene octyl ether (manufactured by TAKEMOTO OIL & FAT Co., Ltd., trade name: "TAKESURF D-1004", HLB value: 11.5, number-average molecular weight: 480)
(D-2): Polyoxyethylene lauryl ether (manufactured by TAKEMOTO OIL & FAT Co., Ltd., trade name: "TAKESURF D-1105", HLB value: 10.8, number-average molecular weight: 510)
(D-3): Polyoxyethylene dodecyl ether (manufactured by TAKEMOTO OIL & FAT Co., Ltd., trade name: "TAKESURF D-1103-D", HLB value: 8.1, number-average molecular weight: 530)
(D-4): Polyoxyethylene stearyl ether (manufactured by TAKEMOTO OIL & FAT Co., Ltd., trade name: "TAKESURF D-1402", HLB value: 4.9, number-average molecular weight: 590)
(D-5): Polyoxypropylene dodecyl ether (manufactured by TAKEMOTO OIL & FAT Co., Ltd., trade name: "TAKESURF D-1106DIR", HLB value: 11.6, number-average molecular weight: 730)
(D-6): Polyethylene glycol monostearic acid ester (manufactured by NIHON EMULSION Co., Ltd., trade name: "EMALEX 810", HLB value: 11, number-average molecular weight: 620)
(D-7): Polyethylene glycol distearic acid ester (manufactured by NIHON EMULSION Co., Ltd., trade name: "EMALEX 600di-S", HLB value: 8, number-average molecular weight: 840)
(D-8): Polyoxyethylene polystyrylphenyl ether (manufactured by TAKEMOTO OIL & FAT Co., Ltd., trade name: "TAKESURF D-6112", HLB value: 10.9, number-average molecular weight: 630)
(D-9): Polyoxypropylene polystyrylphenyl ether (manufactured by TAKEMOTO OIL & FAT Co., Ltd., trade name: "TAKESURF D-6105-W", HLB value: 11.1, number-average molecular weight: 890)
(D-10): Polyoxyethylene cumylphenyl ether (manufactured by TAKEMOTO OIL & FAT Co., Ltd., trade name: "TAKESURF D-7010", HLB value: 13.3, number-average molecular weight: 460)
(D-11): Polyoxyethylene-polyoxypropylene nonylphenyl ether (manufactured by AOKI OIL INDUSTRIAL Co., Ltd., trade name: "BLAUNON NPP-0802R", HLB value: 13.5, number-average molecular weight: 640)
(D-12): Polyoxyethylene naphthyl ether (manufactured by TAKEMOTO OIL & FAT Co., Ltd., trade name: "TAKESURF D-7213-W", HLB value: 12.2, number-average molecular weight: 470)
(D-13): Polyoxyethylene sorbitan oleic acid ester (manufactured by TAKEMOTO OIL & FAT Co., Ltd., trade name: "TAKESURF D-945", HLB value: 15.0, number-average molecular weight: 620)
(D-14): Polyoxyethylene glycerin ether monostearic acid ester (manufactured by NIHON EMULSION Co., Ltd., trade name: "EMALEX GM-10", HLB value: 11, number-average molecular weight: 1,700)
(D-15): Polyoxyalkylene diglyceryl ether (manufactured by TAKEMOTO OIL & FAT Co., Ltd., trade name: "TAKESURF K-17", HLB value: 11.2, number-average molecular weight: 2,700)
(D-16): Polyoxyethylene perfluoroalkyl ether (manufactured by DIC Corporation, trade name: "MEGAFAC F-444", HLB value: 8.5, number-average molecular weight: 630)

All of (D-1) to (D-16) have an acid value of 0 mgKOH/g and an amine value of 0 mgKOH/g.

### Inorganic solid lubricant (S)

(S-1): Graphite (manufactured by Fuji Graphite Works Co., Ltd., trade name: "MF-10N", particle diameter: 10 µm)
(S-2): Graphite (manufactured by Fuji Graphite Works Co., Ltd., trade name: "RCG-1Q", particle diameter: 1.0 µm)
(S-3): Graphite (manufactured by Fuji Graphite Works Co., Ltd., trade name: "RCG-3Q", particle diameter: 3.2 µm)
(S-4): Graphite (manufactured by Fuji Graphite Works Co., Ltd., trade name: "MF-20N", particle diameter: 20 µm)
(S-5): Graphite (manufactured by Fuji Graphite Works Co., Ltd., trade name: "ACG-45J", particle diameter: 45 µm)
(S-6): Graphite (manufactured by Fuji Graphite Works Co., Ltd., trade name: "SC-120", particle diameter: 63 µm)
(S-7): Fluorinated graphite particles (manufactured by Central Glass Co., Ltd., trade name: "CEFBON CMC", particle diameter: 5.0 µm, referred to as "F-graphite" in Tables)
(S-8): Graphene particles (manufactured by EM Japan Co., Ltd., trade name: "Graphene Nanopowder G-12S", particle diameter: 4.5 µm, referred to as "Graphene" in Tables)
(S-9): Carbon nanotube (manufactured by Microphase Co., Ltd., trade name: "TNCNT", particle diameter: 5.0 µm, referred to as "CNT" in Tables)
(S-10): Boron nitride particles (manufactured by Denka Company Limited, trade name: "SP-2", particle diameter: 4.0 µm, referred to as "BN" in Tables)

### Organic solid lubricant (G)

(G-1): Polytetrafluoroethylene particles (manufactured by KITAMURA LIMITED, trade name: "KTL-2N", particle diameter: 3.0 µm, referred to as "PTFE" in Tables)
(G-2): Melamine cyanurate particles (manufactured by Nissan Chemical Corporation, trade name: "MC-6000", particle diameter: 2.0 µm, referred to as "Melamine" in Tables)
(G-3): Benzoguanamine particles (manufactured by Nippon Shokubai Co., Ltd., trade name: "EPOSTAR L15", particle diameter: 9 µm, referred to as "Guanamine" in Tables)

### Solvent

### NMP: N-methyl-2-pyrrolidone

### PX: p-xylene

From the results in Tables above, the following was found.

As shown in Comparative Example 1, when the coating composition containing the solid lubricant, the thermosetting resin, and the solvent did not originally contain a dispersing agent, and the coating layer on the wire surface formed of the coating composition did not contain the dispersing agent, all of the liquid dispersion stability, the bending durability, and the sterilization resistance against hydrogen peroxide gas were inferior. As shown in Comparative Example 2, when the fluorine-containing surfactant was used as a dispersing agent, the liquid dispersion stability was improved as compared with Comparative Example 1, but the bending durability was deteriorated, and the sterilization resistance against hydrogen peroxide gas was slightly improved. As shown in Comparative Example 3, when the nonionic dispersing agent (N) specified in the present invention was contained, but a thermoplastic resin was contained instead of the thermosetting resin, the liquid dispersion stability was improved as compared with Comparative Example 1, but the bending durability and the sterilization resistance against hydrogen peroxide gas were deteriorated.

In contrast, as shown in Examples 1 to 41, all the coating compositions according to the present invention, which contained the nonionic dispersing agent (N) specified in the present invention, had good liquid dispersion stability. Furthermore, the wires having a surface on which a coating layer was formed using such a coating composition had both good bending durability and good sterilization resistance against hydrogen peroxide gas. In particular, in Examples 8 to 12 in which the nonionic dispersing agent (N) had an alkyleneoxy group and an aromatic ring group, the liquid dispersion stability, the bending durability, and the sterilization resistance against hydrogen peroxide gas were better. In Examples 5, 9, and 11 in which the nonionic dispersing agent (N) had a polypropyleneoxy group as an alkyleneoxy group, better sterilization resistance against hydrogen peroxide gas was exhibited. When the thermosetting resin was a polyamideimide resin, the bending durability and the sterilization resistance against hydrogen peroxide gas were better (Example 28 with respect to Example 31).

The present invention has been described together with embodiments thereof. However, we do not intend to limit our invention in any of the details of the description unless otherwise specified. We believe that the invention should be broadly construed without departing from the spirit and scope of the invention as defined by the appended claims.

The present application claims priority to JP2022-156219 filed in Japan on September 29, 2022, the contents of which are incorporated herein by reference as a part of the description.

### Reference Signs List

- 2: electronic endoscope (endoscope)
- 3: insertion part
- 3a: flexible tube
- 3b: bending part (angle part)
- 3c: tip part
- 5: main body operating unit
- 6: universal cord
- 11: force gauge
- 12: lubricating wire
- 13, 15: pulley
- 14: SUS rod
- 16: weight
- A: stroke (10 mm)
- α: angle (100 degrees)

## Claims

1. A coating composition for an endoscope, comprising:
a solid lubricant;
a thermosetting resin;
a solvent; and
a nonionic dispersing agent,
wherein the nonionic dispersing agent is a compound constituted by a carbon element, a hydrogen element, and at least one of an oxygen element or a nitrogen element.

2. The coating composition for an endoscope according to claim 1,
wherein the dispersing agent includes a compound having an alkyleneoxy group.

3. The coating composition for an endoscope according to claim 2,
wherein the compound having an alkyleneoxy group has an aromatic ring group.

4. The coating composition for an endoscope according to claim 2,
wherein the alkyleneoxy group includes a propyleneoxy group.

5. The coating composition for an endoscope according to claim 1,
wherein the dispersing agent has a molecular weight of 100 to 2,000.

6. The coating composition for an endoscope according to claim 1,
wherein the thermosetting resin includes a polyamideimide resin.

7. The coating composition for an endoscope according to claim 6,
wherein the polyamideimide resin includes a structural unit represented by Formula (1) below as a constituent component of polyamideimide, 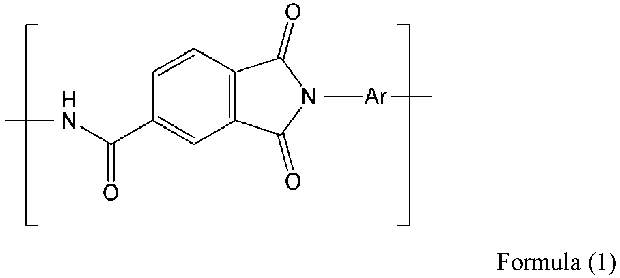
where Ar represents a divalent group having an aromatic ring.

8. The coating composition for an endoscope according to claim 7,
wherein Ar in the Formula (1) represents a phenylene group, a diphenylenemethane group, a diphenylenepropane group, a diphenylene ether group, or a diphenylene amine group.

9. The coating composition for an endoscope according to claim 6,
wherein the polyamideimide resin has a weight-average molecular weight of 10,000 to 100,000.

10. The coating composition for an endoscope according to claim 1,
wherein the solid lubricant includes inorganic particles, and
the inorganic particles include at least one of graphite, fluorinated graphite, graphene, carbon nanotube, or boron nitride.

11. The coating composition for an endoscope according to claim 10,
wherein the inorganic particles have a particle diameter of 0.5 to 70 µm.

12. The coating composition for an endoscope according to claim 11,
wherein the inorganic particles have a particle diameter of 1.0 to 50 µm.

13. The coating composition for an endoscope according to claim 1,
wherein the solid lubricant includes organic particles, and
the organic particles include at least one of fluororesin, melamine cyanurate, or benzoguanamine.

14. The coating composition for an endoscope according to claim 13,
wherein the organic particles have a particle diameter of 0.5 to 30 µm.

15. The coating composition for an endoscope according to claim 14,
wherein the organic particles have a particle diameter of 1.0 to 10 µm.

16. The coating composition for an endoscope according to claim 1,
wherein the solvent includes at least one of N-methyl-2-pyrrolidone, N,N-dimethylacetamide, N,N-dimethylformamide, hexamethylphosphoric triamide, γ-butyrolactone, or 1,3-dim ethyl -2-imi dazoli dinone.

17. A lubricating member for an endoscope,
wherein the lubricating member is subjected to a coating treatment with the coating composition for an endoscope according to any one of claims 1 to 16.

18. The lubricating member for an endoscope according to claim 17,
wherein the lubricating member for an endoscope is a lubricating wire for an endoscope.

19. A method for manufacturing a lubricating member for an endoscope, the method comprising:
applying the coating composition for an endoscope according to any one of claims 1 to 16 onto a surface of a member for an endoscope.

20. A flexible tube for an endoscope, comprising the lubricating member for an endoscope according to claim 17.

21. An endoscope comprising the flexible tube for an endoscope according to claim 20.
